# EUROPEAN PATENT SPECIFICATION

(11) **EP 1 842 921 B1**
(45) Date of publication and mention of the grant of the patent: **15.06.2011**
(21) Application number: 05700484.8
(22) Date of filing: 26.01.2005
(51) Int. Cl.: C12N 15/861, C12N 15/31, A61K 48/00, A61P 35/00

(54) **RECOMBINED ADENOVIRUS P53 PREPARATION FOR TREATING TUMOR**
PRÄPARATION VON REKOMBINIERTEM ADENOVIRUS-P53 ZUR TUMORBEHANDLUNG
PREPARATION D'ADENOVIRUS P53 RECOMBINE DANS LE TRAITEMENT D'UNE TUMEUR

(43) Date of publication of application: 10.10.2007
(73) Proprietor: Peng, Zhaohui, c/o SiBiono Co., Shenzhen, Guangdong 518057 (CN); Zhang, Xiaozhi, Shenzhen, Guangdong 518057 (CN)
(72) Inventor: Peng, Zhaohui, c/o SiBiono Co., Shenzhen, Guangdong 518057 (CN); Zhang, Xiaozhi, Shenzhen, Guangdong 518057 (CN)
(74) Representative: Grünecker, Kinkeldey, Stockmair & Schwanhäusser Anwaltssozietät
(86) International application number: PCT/CN2005/000111
(87) International publication number: WO 2006/079244

(56) References cited:
- CN-A- 1 401 778
- CN-C- 1 079 833
- CN-C- 1 177 057
- PENG ZHAHUI: "Current status of gendicine in China: Recombinant human Ad-p53 agent for treatment of cancers" HUMAN GENE THERAPY, MARY ANN LIEBERT, NEW YORK ,NY, US, vol. 16, no. 9, September 2005 (2005-09), pages 1016-1027, XP002406056 ISSN: 1043-0342
- PENG ZH: "The genesis of Gendicine: The story behind the first gene therapy" BIOPHARM INTERNATIONAL, 1 May 2004 (2004-05-01), XP002461874 Advabstar Communications Inc
- ANONYMOUS: "Milestone" SIBIONO, [Online] XP002461875 Retrieved from the Internet: URL:http://www.sibiono.com/en/index.asp?Ca talogID=48> [retrieved on 2007-12-11]
- SWISHER S.G. ET AL.: CLIN. CANCER RES., vol. 9, January 2003 (2003-01), pages 93-101,
- NEMUNAITIS J. ET AL.: J. CLIN. ONC., vol. 18, no. 3, 2000, pages 609-622,
- PENG Z.: "The genesis of Gendicine: The story behind the first gene therapy." BIOPHARM INTERNATIONAL, no. 3536, May 2004 (2004-05), Advanstar Publications

## Description

### FIELD OF THE INVENTION

The present invention relates to uses of recombinant adenovirus expressing p53 for improving the life quality of individuals suffering from tumor and reducing side effects caused by anti-tumor chemotherapy and anti-tumor radiotherapy that these individuals are subjected to.

### BACKGROUND OF THE INVENTION

Tumors are a leading cause of death in animals and humans. Common forms of tumor therapy today include surgery, radiotherapy and chemotherapy. In spite of advances in the field of tumor treatment, each of these known therapies has serious side effects. For example, surgery disfigures the patient or interferes with normal bodily functions. Chemotherapy or radiotherapy may cause patients to develop acute debilitating symptoms including nausea, vomiting, diarrhea, hypersensitivity to light, hair loss, etc. The side effects caused by those cytotoxic compounds frequently limit the frequency and dosage at which they can be administered.

Indications of these side effects can be demonstrated through biochemical and physiological tests such as white blood cells (WBC), hemoglobin (Hb), aspartate aminotransferase (AST), alanine aminotransferase (ALT). The results of these tests indicate that the biochemical and physiological parameters of patients subjected to chemotherapy and radiation therapy reach to a lower level as compared to those of the control patients which do not receive tumor treatment. Often times, tumor patients suffer greatly from these side effects when they are subjected to chemotherapy or radiation therapy. In some cases, to the patient, the pain and suffering derived from these side effects are even greater than that of the cancer itself.

Nowadays, many attempts have been made to reduce the severe side effects of these cancer therapies.

United States Patent Number 6,479,500 describes the structure and effectiveness of a chemical agent, which can reduce the side effects caused by anti-tumor agent.
United States Patent Number 5,017,371 describes the use of interferon in reducing the toxic side effects associated with radiation therapy or chemotherapy.

United States Patent Number 6,462,017 describes methods of reducing the severity of side effects in tumor patients using thymosin alpha in combination with chemotherapy.

Thus, it is clear that there exists a great need to develop an effective and novel method to reduce side effects of tumor therapies in order to improve the life quality of tumor patients and help them to withstand the whole course of either chemotherapy or radiation therapy.

### SUMMARY OF THE INVENTION

According to the present invention, the administration of a recombinant adenovirus expressing p53 in an effective amount in combination with traditional tumor therapies could effectively reduce the side effects of the tumor patients when they are subjected to traditional treatments such as chemotherapy and radiotherapy. The alleviation of side effects would improve the life quality of those patients subjected to chemotherapy or radiotherapy. The testing results of the biological, physiological and biochemical parameters can prove the improvement in patients.

The present invention concerns the use of a treatment-effective amount of a recombinant virus expressing p53 as mentioned below in section 4 for the preparation of a pharmaceutical composition for:
(a) ameliorating pain, Karnofsky Performance Status scoring, or low white blood cell count (leukocytopenia) caused by anti-tumor chemotherapy; or
(b) ameliorating leukocytopenia caused by anti-tumor radiotherapy.

The pharmaceutical composition is to be administered prior to the administration of the chemotherapy or radiotherapy, typically on any day of the 3 days preceding the day of radio- or chemotherapy, or on the day before the day of radio- or chemotherapy, or on the same day of radio- or chemotherapy. In an alternative embodiment, the pharmaceutical composition is to be administered simultaneously with radio- or chemotherapy.

The administration of a recombinant adenovirus expressing p53 alone can also improve the life quality of tumor patients. As indications of such improvements, the tumor patients may be observed to have better appetite, better sleep, higher level of energy, less pain, and desired weight gain.

### BRIEF DESCRIPTION OF THE FIGURES

In order to further describe the features, advantages, objectives and other aspects of the present invention, the detail content of the above summary will be illustrated accompanying with the figures, which belong to the part of the invention. However, it should be understood that these figures are only illustrative and therefore are not intended to limit the scope of the invention.
Fig. 1 shows a schematic process of the construction of a recombinant p53 adenovirus described herein.
Fig. 2 shows a flow chart of the production protocols of a recombinant p53 adenovirus.
Fig. 3 shows a graph of an agarose gel electrophoresis of a PCR amplification product of p53 gene form the recombinant p53 adenovirus after generations of passage, using p53 cDNA as template, and 5'CCACGACGGTGACACGCTTC, and 5'CAAGCAAGGGTTCAAAGAC as primers, illustrating the stability of the recombinant p53 adenovirus. A DNA fragment of 1400bp was obtained after PCR amplification of p53 gene. Lane 1: DNA molecular weight standard markers; Lanes 2, 3, 4: Results of the PCR amplification of p53 cDNA.
Fig. 4 shows the Western Blot results of a recombinant p53 adenovirus, extracted from lysed cells transfected with the recombinant p53 adenovirus 36 hours earlier. The transfected cells were human laryngeal cancer cells Hep-2, and non-small cell cancer cells H1299. Lane 1: Protein molecular weight standard markers; Lanes 2 and 3: Negative controls of Hep-2 cells and H1299 cells, both not transfected with recombinant p53 adenovirus (Ad-p53), respectively; Lanes 4 and 5: Hep-2 cells and H1299 cells transfected with recombinant p53 adenovirus (Ad-p53), respectively.
Fig. 5 shows a curve demonstrating the killing effect of a recombinant p53 adenovirus on Hep-2 cells. Hep-2 cells were cultured on six-well plates until their density reaches 1x10⁶ per well. Ad-p53 was administered to transfect the cells at 100 MOI for different time periods (24, 48, 72, and 96 hours). Cells were stained with Trypan Blue and the percentage of blue cells (dead cells) was then counted.

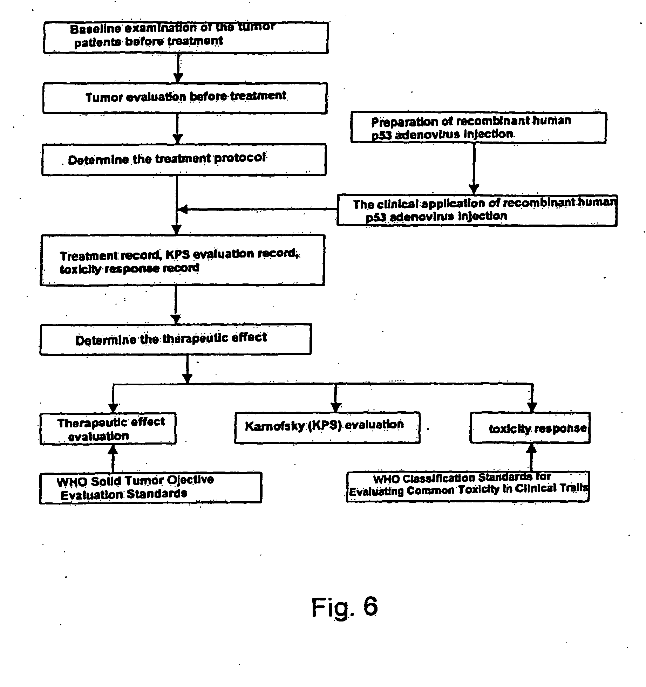

Fig. 6 shows a flow chart of procedures of clinical observation and methods of evaluating the effects of the recombinant p53 adenovirus on tumor treatments.

### DETAILED DESCRIPTION OF THE INVENTION

In the clinical trials, various cancers such as head and neck squamous cancer, non-small cell cancer, liver cancer, lung cancer, thyroid cancer, cervical cancer, etc. were cured by administering a recombinant adenovirus expressing p53 to patients. Independent of its abilities of suppressing tumors, it was discovered that recombinant adenovirus expressing p53 is also able to reduce the side effects caused by conventional chemotherapy and radiation therapy and improve the life quality of tumor patients. Below, we first describe the construction of the recombinant p53 adenovirus and the clinical application thereof The selected recombinant p53 adenovirus in the embodiments is a commercially available product with the trade name Gendicine.

### 1. p53 and p53 Mutations in Cancer

P53 is currently recognized as a tumor suppressor gene (Montenarh, M., 1992). Elevated expression level has been found in many cells transformed by chemical carcinogenesis, ultraviolet radiation, and several viruses, including SV40 (simian virus 40). The p53 gene is a frequent target of mutational inactivation in a wide variety of human tumors and is already documented to be the most frequently-mutated gene in human tumor cells up to now (Mercer, 1992). p53 mutation occurs in more than 50% of non-small cell lung cancer (Hollestein et al., 1991).

The p53 gene encodes a phosphorylated protein with 393-amino-acid residues that can bind large-T antigen and E1B protein. The expression of p53 protein is found in normal tissues and cells, but at lower level than that in transformed cells or tumor cells. Interestingly, wild-type p53 plays an important role in the regulation of cell growth and division. Over-expression of wild-type p53 has shown anti-proliferative function in some tumor cell lines. Therefore, p53 can act as a negative regulator of cell growth (Weinberg, 1991) and may directly suppress uncontrolled cell growth or indirectly inhibit the uncontrolled growth by activating downstream target genes. Thus, absence or inactivation of wild type p53 may contribute to permanent transformation. However, some studies indicate that the presence of mutant p53 may be necessary for the full expression of the transforming potential of the gene.

Wild-type p53 is recognized as a very important growth regulator in many cell types due to the role of its genetic and biochemical traits. Mis-sense mutations are common for the p53 gene and are essential for the transforming ability of the oncogene. A single genetic change prompted by point mutations can create carcinogenic p53. Unlike other oncogenes, however, p53 point mutations are known to occur in at least 30 distinct codons, and are dominant mutations that can produce shifts in cell phenotype without accumulating to homozygote mutations. Additionally, many of these dominant negative alleles appear to be tolerated in the organism and passed on in the germ line. Various mutant alleles of p53 have been found with functional changes ranging from minimally dysfunctional to dominant (Weinberg, 1991).

Casey and colleagues have reported that transfection of DNA encoding wild-type p53 into two human breast cancer cell lines restores growth suppression control in such cells (Casey *et al.,* 1991). A similar effect has also been demonstrated on the transfection of wild-type p53, but not mutant p53 into human lung cancer cell lines (Takahashi*, et al.,* 1992). The wild-type p53 appears dominant over the mutant gene and will inhibit specifically against cell proliferation when transfected into cells with mutant p53 gene. Normal expression of the transfected wild-type p53 is observed in cells with endogenous p53 and does not affect the growth of such cells. Thus, such constructs according to the present invention may be taken up by normal cells without adverse effects.

It is thus possible to treat the tumor related to p53 mutation with wild type p53 to reduce the number of malignant cells. However, the above-mentioned studies are far from achieving such a goal due to at least a reason that DNA transfection cannot be employed to introduce DNA into tumor cells in a patient *in vivo.*

### 2. Gene Therapy Approaches

There have been several experimental approaches proposed to gene therapy to date, but each is proved to have their particular drawbacks (Mulligan, 1993). In the above-mentioned basic transfection method, DNA fragment containing the gene of interest is introduced into cells non-biologically, for example, by permeabilizing the cell membrane physically or chemically. Accordingly, this approach is limited to treat cells such as lymphocytes, which can be temporarily removed from the body and can tolerate the cytotoxicity caused by the treatment. Liposomes or protein conjugated with certain lipids and amphophilic peptides can be used for transfection, but the efficiency of gene integration is still very low, on the order of one integration event per 1,000 to 100,000 cells, and expression of transfected genes is often limited to days in proliferating cells or weeks in non proliferating cells. DNA transfection is clearly, therefore, not a suitable method for tumor treatment.

A second approach employs the natural ability of viruses entering cells and introduces target gene into cells with some of their own genetic material. Retrovirus have been considered as an expected gene delivery vector due to its ability of integrating his genes into the host genome DNA, carrying a large amount of foreign genetic material, infecting a broad spectrum of species and cell types and using packaging cell lines to perform the production and packaging thereof . However, three major problems hamper the practical use of retrovirus. First, retroviral infectivity depends on the availability of the viral receptors on the surface of target cells. Second, retroviruses only integrate efficiently into replicating cells. And finally, retroviruses are difficult to concentrate and purify.

### 3. Construction of adenovirus for Gene Therapy

Human adenoviruses are double-stranded DNA viruses with genome sizes of approximate 36 kb (Tooza. 1981). As a model system for the research of eukaryotic gene expression, adenoviruses have been widely studied and well characterized, which makes them an attractive candidate system for the development of a gene transfer system. This group of viruses is easy to grow and manipulate, and they exhibit a broad host range in vitro and in vivo. In lytically infected cells, adenoviruses are capable of shutting off host protein synthesis, directing cellular machineries to synthesize large quantities of viral proteins, and replicating copious amounts of virus.

The E1 region of the adenoviral genome includes E1A and E1B genes which encode proteins responsible for the transcription regulation of the viral genome, as well as a few genes of host cells. E2 region includes E2A and E2B genes and its encoded protein products such as DNA-binding protein, DNA polymerase and a terminal protein (TP protein) as a primer for replication are responsible for the viral replication. E3 gene products involve immune escape of adenoviruses and prevent host cells form being attacked by cytotoxic T cells and resulting breakdown, thus playing an important role in the viral survival and viral propagation. In summary, the functions of various E proteins (early protein) are associated with DNA replication, late gene expression, and shutoff of some function of host cells. The late gene products include most of the virion capsid proteins, which are expressed only after the replication of adenoviruses is substantially completed and primed by the major late promoter (MLP). MLP exhibits high efficiency of initiating transcription during the late phase of the adenoviral infection (Stratford-Perricaudet and Perricaudet, 1991a).

As only a small portion of the viral genome is required in cis-acting (i.e., necessary for the replication and packaging of adenovirus) (Tooza, 1981), adenovirus-derived vectors can offer insertion space for the substitution of large DNA fragments when produced in 293 cells. Ad5-transformed human embryonic kidney 293 cell line (Graham, et al., 1977) is capable of providing trans-acting elements for the replication and packaging of the adenoviral vectors. Therefore, the characteristics of adenovirus render them good candidates for tumor gene therapy *in vivo* (Grunhaus and Horwitx, 1992).

Particular advantages of an adenovirus system for delivering foreign proteins to a cell include that: (i) It has a ability to substitute relatively large pieces of viral DNA by foreign DNA; (ii) The structure of recombinant adenovirus is stable; (iii) It is safe for injecting adenovirus to humans; and (iv) There is not any known association of adenoviral infection with cancer or malignancies; (v) Adenovirus can be produced with high titers; and (vi) Adenovirus possesses high infectivity.

Compared to retroviral vector, adenoviral vector has high level of exogenous gene expression and adenovirus replication is independent of host gene replication. Because transformed genes in the E1 region of adenovirus can be readily deleted and has no effect on the efficient expression of exogenous genes, oncogenic risk from adenovirus vector is thought to be negligible (Grunhaus & Horwitz, 1992).

In general, adenovirus gene transfer systems is a recombinant adenovirus engineered by gene engineering which is rendered replication-incompetent by deletion of E1 region of its genome and yet still retains its competency for infection. Relatively large exogenous genes can be expressed when further deletions are made in the adenovirus genome. For example, adenoviruses deleted in both E1 and E3 regions are capable of carrying up to 10 Kb of exogenous DNA and can be grown to high titers in 293 cells (Stratford-Perricaudet and Perricaudet, 1991 a). Surprisingly persistent expression of exogenous genes following adenoviral infection has also been reported.

Adenovirus-mediated gene transfer has recently been evaluated as a means of mediating gene transfer into eukaryotic cells and experimental animals. For example, in treating mice suffered from ornithine transcarbamylase (OTC) deficiency which is a rare recessive genetic disorder, it was found that adenoviral vector could be employed to deliver the normal OTC enzyme gene. Unfortunately, the expression of OTC was only achieved in 4 out of 17 instances (Stratford-Perricaudet et al., 1991b). Therefore, the defect was only partially corrected in most of the experimental mice and led to no physiological or phenotypic change. These results certainly offer little encouragement for the use of adenoviral vectors in tumor therapy.

The study of using adenovirus to transduce the gene for cystic fibrosis transmembrane conductance regulator (CFTR) into the pulmonary epithelium of rats for the treatment of cystic fibrosis has only been partially successful, although it has not been possible to assess the biological activity of the transferred gene in the epithelium of the animals (Rosenfeld et al., 1992). Again, these studies demonstrated that the expression of the CFTR protein in lung airway cells showed no physiologic effect.

These results do not demonstrate that adenovirus is able to direct enough exogenous expression in infected cells to achieve a physiological effect, and the researchers do not, therefore, suggest a use of the adenovirus system in tumor therapy. Furthermore, prior to the present invention, it was commonly thought that p53 could not be incorporated into a packaging cell used in the preparation of recombinant adenovirus, as it would be toxic to packaging cells. As an early expressed protein E1B of adenovirus binds to p53 protein, it was thought to be a further technical reason why adenoviral vector and p53 gene could not be combined together.

### 4. Construction of Ad-p53 and tumor suppression

The present invention provides a new use of an effective tumor suppressor and vector for tumor gene therapy. This recombinant virus exploits the advantages of adenoviral vectors, such as high titer, broad target range, efficient transduction, and non-integration in target cells. Specifically, the present invention provides a new use of a replication-defective, helper virus-independent adenovirus that expresses wild type p53 (Ad5RSV-p53) under the control of the Rous Sarcoma Virus promoter.

Control functions on expression vectors are often provided from viruses when the expression of exogenous genes is required in mammalian cells. For example, commonly used promoters are derived from polyoma, adenovirus 2 and simian virus 40 (SV40). The early and late promoters of SV40 virus are particularly useful because both are obtained easily from a fragment of the virus which also contains the replication origin of SV40 virus. Smaller or larger SV40 fragments may also be used provided there is included the approximately 250 bp sequence extending from the HindIII site toward the BglI site located in the replication origin. This 250bp sequence may be retained or deleted as necessary. Further, it is also possible, and often desirable, to utilize promoter or control sequences normally associated with the selected target gene, provided such control sequences are compatible with the host cell systems.

A replication origin may be provided by an exogenous origin derived form SV40 or other sources (e.g., polyoma, adenovirus, herpetic stomatitis virus VSV, bovine papilloma virus BPV), or may be provided by the host cell chromosomal replication mechanism. If the vector is integrated into the host cell chromosome, the latter is often sufficient.

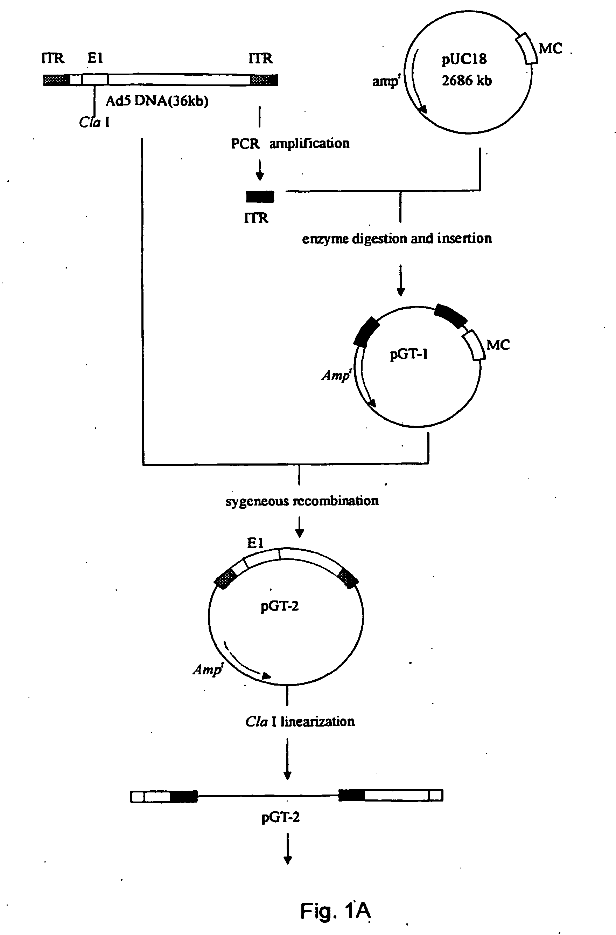

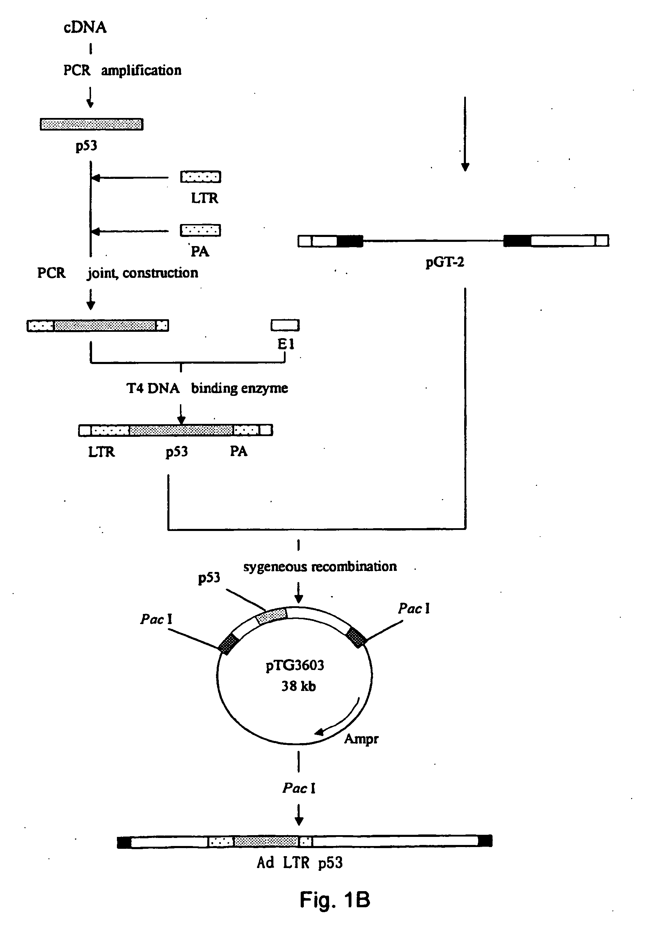

The design and construction of a recombinant p53 adenovirus is illustrated in Fig. 1. In connection with this, an improved protocol has been developed for constructing and identifying such recombinant adenovirus. After identification, the p53 recombinant adenovirus was structurally confirmed by the PCR analysis. After isolated and confirmed structurally, the p53 adenovirus was used to infect human laryngeal cancer Hep2 cell and non-small lung cancer H1299 cell which has knocked out a homologous p53 gene. The results of Western blot showed that the exogenous p53 protein was expressed at a high level.

The studies disclosed herein indicate that a recombinant adenovirus expressing p53 possesses properties of tumor suppression, which appear to operate by restoring p53 protein function in tumor cells. These results may support the use of the Ad5RSV-p53 virion as a therapeutic agent for tumor therapy.

As a new gene transfer system, recombinant adenovirus has many potential applications in gene therapy and the development of vaccines. The propagation of recombinant adenovirus is therefore an important molecular biological tool. The existing methods for propagating recombinant adenovirus include the transfection of 293 cell mediated by phosphate calcium precipitation and subsequent plaque analysis on transfected cells. The transfection efficiency associated with this method needs to be improved and its procedures need to be simplified.

### 5. Improved Protocol for ameliorating the side effects caused by chemotherapy and radiotherapy

Treatment of tumor has, over the last twenty years, been the focus of a significant research and development effort. Many approaches to tumor therapy have been investigated. As a practical matter, tumor therapies involve use of multiple treatment methods including surgical excision, radiotherapy, chemotherapy, bone marrow transplantation (for treating patients with some types of hematological malignancies, particularly acute granulocyte leukemia), and Chinese traditional herb medicine and the like. The specific protocol utilized to treat a given malignancy, depends on the nature, location and type of malignancy being treated. Surgical excision is still the most preferred method for treatment of tumors and is used as a main therapy for about 60% of tumors. However, surgical excision is often combined with radiation therapy and/or chemotherapy to complete a whole treatment protocol. In cases where the malignancy is not localized or where its location lowers the probability of successful removal or excision by surgical techniques, chemotherapy and radiation therapy are often used in combination.

Chemotherapy has been demonstrated to effectively treat with some types of cancer, including Hodgkin's disease, acute lymphocyte and granulocyte leukemia, testicular cancer, non-Hodgkin's lymphoma and the like. In other types of tumor, chemotherapy has been used effectively to decrease the size of tumors prior to surgery. Chemotherapy often involves the use of combinations of chemotherapeutic agents. New protocols are being developed and tested continuously by the medical research community.

However, anti-tumor agents are drugs which, in addition to killing tumor cells, can damage normal tissue. Even with extensive researches that have been conducted to define dosage levels and scheduling of drug administration, chemotherapy often results in unpleasant and possibly dangerous side effects due to drug toxicity. Radiation therapy also produces the similar problems. Most common of such side effects are nausea, vomiting, alopecia, and bone marrow depression. Such side effects are usually, but not always, reversible. Some anti-cancer drugs may permanently damage the nervous system, heart, lungs, liver, kidneys, gonads or other organs. Some chemotherapeutic agents are themselves carcinogenic. Patients subjected to radiotherapy or chemotherapy must also take prophylactic agents or prophylactic measures simultaneously to avoid the life-threatening infections resulted form the immuno-suppressed condition induced by the anti-cancer therapy.

Some treatment strategies have been developed to counteract the side effects of radiotherapy and chemotherapy for tumors. For example, drugs can be administered to provide some relief from nausea, the administration of antibiotics or dwelling in laminar air-flow wards can help to fight infection, and transfusions can be administered to increase blood cell and platelet counts, and the like.

Chemotherapeutic agents can be classified broadly and play an important role in treating tumors in humans. Such chemotherapeutic agents include, but not only limit to, alkyleting agents (e.g., nitrogen mustards), antimetabolites (e.g., pyrimidine analogs), radioactive isotopes (e.g., phosphorous and iodine), hormones (e.g., estrogens and adrenocorticosteroids), miscellaneous agents (e.g., hydroxyurea) and natural products (e.g., pacilitaxel, vincaleukoblastine and antibiotics). Although the preceding compounds are not curative agents, they are widely recognized in the medical profession as useful in the suppression, palliation, retardation and control of malignant tumors. While these compounds have been found to be effective and are in general clinical use as antiproliferative agents, there are well recognized drawbacks associated with their administration as anticancer agents. The alkylating agents have marked cytotoxic action and the ability of these drugs to interfere with the mitosis and proliferation of normal cell can be lethal. The antimetabolities can lead to anorexia, progressive weight loss, depression, and coma. Prolonged administration of antimetabolites can result in serious suppression in bone marrow. Both the alkylating agents and the antimetabolities generally have a depressive effect on the immune system. Prolonged administration of natural products such as vincaleukoblastine can also result in bone marrow depression. Hydroxyurea and other chemical derivatives can lead to rapid reduction in levels of adrenocorticosteroids and their metabolites. The administration of hormonal compounds or radioactive isotopes is also undesirable from the viewpoint of inflicting damage on the immune system and thereby disabling the body's defenses against common infections. In most instances, it would be preferable to employ a chemotherapeutic agent which is effective in controlling, retarding, or suppressing the malignant tumors while simultaneously stimulating and promoting the immune function of patients.

Chemotherapy agents may be given either in a single or in several large doses or, more commonly, may be given in small doses 1 to 4 times per day and continue to several weeks or months. There are many cytotoxic agents used to treat cancer, and their mechanisms of action are generally poorly understood.

Irrespective of the mechanism, useful chemotherapeutic agents are known to injure and kill cells of both tumors and normal tissues. The successful use of chemotherapeutic agents to treat tumor depends upon the differential killing effect of the agent on tumor cells compared to its side effects on critical normal tissues. Among these effects, the killing of hematopoietic cells and white blood cells can lead to infection. Acute and chronic bone marrow toxicities are also major factors of limiting the use of chemotherapeutic agents in the treatment of tumor. They are both related to a decrease in the number of hemopoietic cells (e.g., pluripotent stem cells and other progenitor cells) caused by both a lethal effect of cytotoxic agents or radiation on these cells and such effects result in the depletion of more mature marrow components, promoting the differentiation of stem cells by a feed-back mechanism and thus decreasing the amount of stem cells. (U.S. Pat. No. 5,595,973) Stimulators and inhibitors of bone marrow kinetics play a prominent role in the induction of damage and recovery patterns (Tubiana, et al., Radiothereapy and Oncology, 29: 1, 1993).

Prevention of, or protection from the side effects of chemotherapy would generate great benefit to tumor patients. However, many efforts for reducing these side effects are not successful. For life-threatening side effects, efforts have concentrated on altering the dose and schedules of the administration. Other options are becoming available, such as the use of granulocyte colony stimulating factor (G-CSF), granulocyte-macrophage-CSF (GM-CSF), epidermal growth factor (EGF), interleukin 11, erythropoietin, thrombopoietin, megakaryocyte development and growth factor, pixykines, stem cell factor, FLT-ligand, as well as interleukins 1, 3, 6, and 7, to increase the number of normal cells in various tissues before the start of chemotherapy (See Jimenez and Yunis, Cancer Research, 52: 413-415; 1992). The mechanisms of protection by these factors, while not fully understood, are most likely associated with an increase in the number of normal critical target cells before chemotherapy, and not with increased survival capability of cells following chemotherapy.

Acute myelosuppression often occurs as a consequence of cytotoxic chemotherapy and is well recognized as a dose-limiting factor in tumor treatment. (U.S. Pat. No. 5,595,973). Although other normal tissues may be adversely affected, bone marrow is particularly sensitive to the antiproliferation-specific treatment such as chemotherapy or radiotherapy. For some tumor patients, hematopoietic toxicity caused by chemotherapeutic agents frequently limits the opportunity for chemotherapy dose escalation. Repeated or high dose cycles of chemotherapy may be responsible for severe stem cell depletion leading to serious long-term hematopoietic hypofunction and marrow exhaustion.

The present invention concerns the use of a treatment-effective amount of a recombinant virus expressing p53 as mentioned above in section 4 for the preparation of a pharmaceutical composition for:
(a) ameliorating pain, Karnofsky Performance Status scoring, or low white blood cell count (leukocytopenia) caused by anti-tumor chemotherapy; or
(b) ameliorating leukocytopenia caused by anti-tumor radiotherapy.

The uses disclosed in the present invention are suitable for use in combination with chemotherapeutic agents. The chemotherapeutic agents may be any kinds of medicine including, but not limited to, cyclophosphamide, taxol, 5-fluorouracil, cisplatinum, methotrexate, cytosine arabinoside, mitomycin C, prednisone, vindesine, carbaplatinum, and vincristine. Among others, the cytotoxic agent may also be an antiviral compound which is capable of destroying proliferating cells. For a general discussion of cytotoxic agents used in chemotherapy, see Sathe, M. et al., Cancer Chemotherapeutic Agents: Handbook of Clinical Data (1978).

The uses of the invention are also particularly suitable for those patients in need of repeated or high doses of chemotherapy or radiotherapy. For some tumor patients, toxicity caused by chemotherapeutic agents frequently limits the opportunity for chemotherapy dose escalation. Repeated or high dose cycles of chemotherapy may be responsible for severe stem cell depletion leading to severe long-term hematopoietic hypofunction and marrow exhaustion. The usesof the present invention provide reduced mortality and improved blood cell count when used in combination with chemotherapy.

The active preparation (refers to a recombinant adenovirus expressing p53 herein) can be administered by any suitable routes, including but not limited to, intra-tumor topical injection, intraperitoneal injection, intra-bladder infusion, intrabronchial instillation, liver arterial injection and peripheral vein instillation, etc. All administration methods are safe and effective. The currently recommended administration method is intro-tumor topical injection. The common vectors, adjuvants and excipients in pharmaceutical area can be used in the formulations of topical injection.

The active preparation can be formulated into solid forms (granule, powder or suppository, etc.) or liquid forms (solution, suspension or emulsion, etc.) and can dissolve in various solutions, which should be aseptic and contain enough peptides, be harmless in the recommended applications, and be very stable under these conditions, but can be degraded by strong acids or strong bases.

The dosage regimen of the active preparation depends on many factors, such as the type of the tumor, the age, weight, sex, the general health of the subject, severity of the patient, the administration route and the formulation of medicine, etc.

Intra-tumor topical injection is most preferred, with the dosage range of from 1×10⁷ VP to 7×10¹³ VP each time (the maximum tolerate dosage is not determined). Currently, 1×10¹²

VP each time per week is usually used in clinical experiments. This dosage is enough for the preparation to produce the maximum treatment effect with the minimum dosage of the agonist. This regimen may minimize the treatment cost and potential toxic side effects.

The pharmaceutical composition is to be administered prior to the administration of the chemotherapy or radiotherapy, typically on any day of the 3 days preceding the day of radio- or chemotherapy, or on the day before the day of radio- or chemotherapy, or on the same day of radio- or chemotherapy. In an alternative embodiment, the pharmaceutical composition is to be administered simultaneously with radio- or chemotherapy.

In the most preferred embodiment, the topical injection with the dosage of 1×10¹² VP each time per week can be applied 72 hours before chemotherapy or radiotherapy. In other embodiments, the preparation can be repetitively applied with multi-courses of treatment. It is preferred that the blood count of the patient (white blood cells, platelets, and neutrophil cells) recover to the level that is suitable for the next chemotherapy treatment (determined by the physician), and the next course should start immediately after the previous course has completed.

In addition to the active ingredients, the pharmaceutical composition may further contain suitable pharmaceutically-acceptable carriers comprising excipients and auxiliaries which facilitate processing of the active compounds into applicable formulation. Further details on techniques for preparation may be found in the latest edition of Remington's Pharmaceutical Sciences (Maack Publishing Co., Easton, Pa.).

The following examples are further descriptions and illustrations for the present invention, which should be construed to limit the scope of the invention.

### EXAMPLES

### Example One. Preparation of Gendicine, a recombinant p53 adenovirus preparation.

A recombinant p53 adenovirus was prepared and used in the clinical trials. The trade name of the recombinant adenovirus is "Gendicine". The detailed process describing the preparation of Gendicine is disclosed in Chinese Patent No.: ZL 02115228.4.

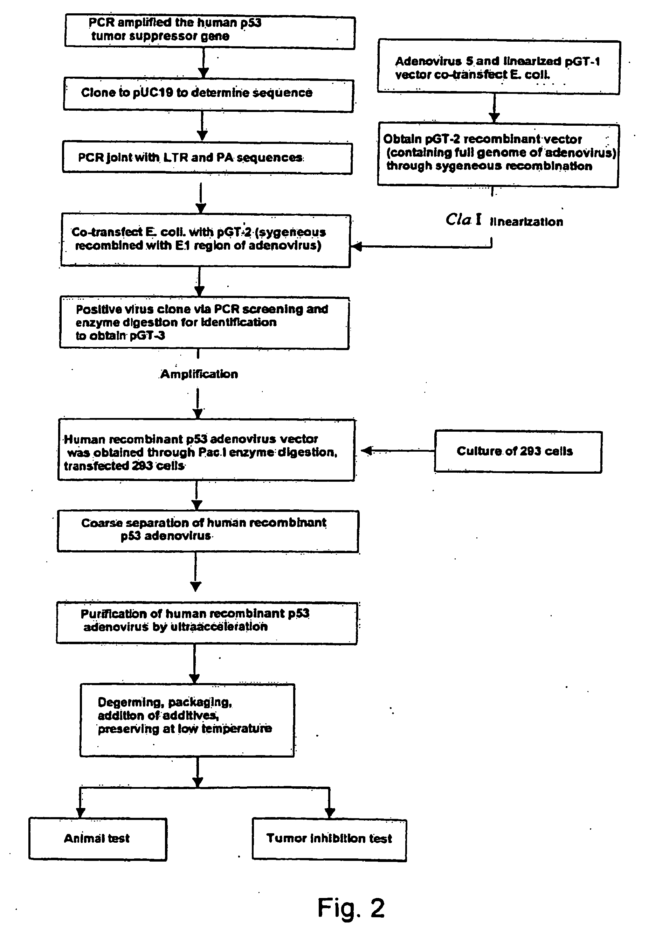

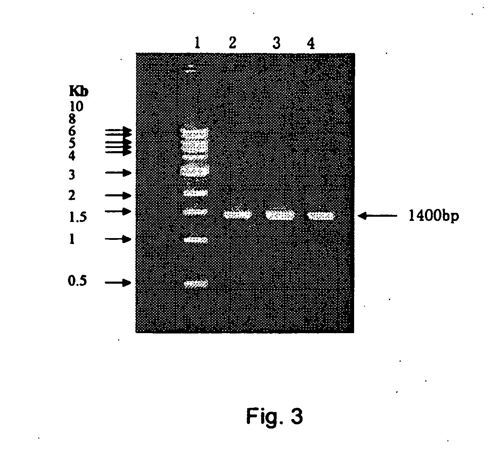

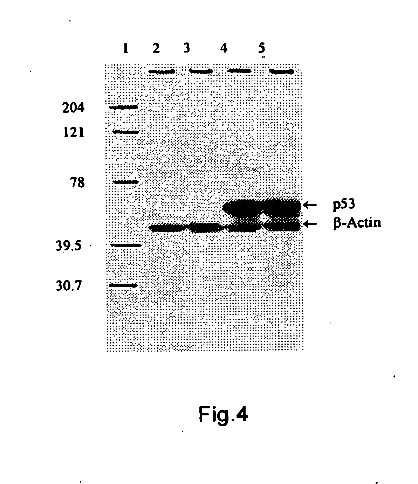

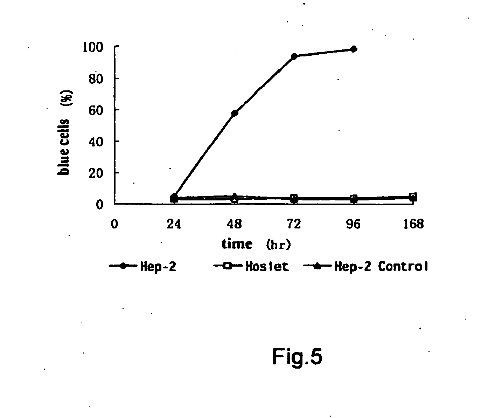

Human recombinant p53 adenovirus was constructed and produced as described in Figure 1 and 2. Adenovirus vector and shuttle vector containing p53 gene were introduced together into E. coli and such recombinant adenovirus was selected to obtain positive recombinant clones. The recombinant clones were amplified in 293 cells, and through multiple steps of purification, clinical grade human recombinant p53 adenovirus was harvested. Fig. 3 shows the results of determination of the structural stability of the recombinant human p53 adenovirus. The result in Fig. 3 shows that after many generations, the recombinant adenovirus maintained its structural stability. Fig. 4 shows the Western Blot results indicating that human recombinant p53 adenovirus can be highly expressed in laryngeal cancer cells Hep-2, and non-small lung cancer cells H1299. Fig. 5 is a graph showing the killing effect of the recombinant p53 adenovirus on Hep-2 cells. Cells were stained with Trypan Blue and the percentage of blue cells (dead cells) was then counted with the results shown in Figure 5.

### Example Two

Material: Recombinant human p53 adenovirus injection (Gendicine) was provided by Shenzhen Sibiono Gene Tech Co. in Shenzhen, China, Batch No. was #SO10731, and the medication was packaged in 1x10¹² VP/Each dose, and each dose was contained in a 2ml size Saline Bottle. There was one bottle in each box.

The clinical trial was designed as multi-center, concurrent control, single-agent, open label, randomized clinical trial.

The selection process for patients was as follows: The patient signed consent form to participate in clinical trials. By pathological analysis, it was confirmed that the patients indeed had suffered form head and neck tumor. The tumor is then classified according to the TNM Classification method. The patients admitted into the trial possessed measurable tumor lesions that were readily observable, and accessible for local injection of drugs. The patient ages ranged from 18 years old to 70 years old, and their life expectancy was at least 3 months. Both male and female patients may be admitted.

Exclusion criteria were chosen as follows: Patients suffered from acute upper respiratory infection, or those patients having uncontrolled fever derived from such infection were excluded. Patients were also excluded if the lesions observed were being treated with other locally-administered medication. Those patients with serious heart, liver, lung, or kidney diseases, tendency of hemorrhage, patients who were pregnant women or nursing mothers were also excluded.

During the trials, patients were withdrawn if they met the following standards: patients who could not bear the high fever caused by using the tested medicine; patients who must stop the trial because of the onset of unexpected toxic side effects; and those patients who withdrew from the trials for personal reasons. These clinical trials lasted from June 2001 to May 2003 in four hospitals, together with 155 subjects.

The clinical trial protocol was designed as follows:

### 1. Group of Gene Therapy combined with Radiotherapy

Each Friday, Gendicine was injected inside of the tumor, at a dose of 10¹² VP/each injection. Three days after the injection, radiotherapy is performed on the patient. The radiation is done using standard or intensity modulated 3-dimensional conformal radiation therapy method, with dosage of 70Gy/35f for about 7-8 weeks. After five weeks and cumulative dosage of 40Gy, the first cycle is completed. After eight weeks of cumulative dosage of 70Gy, the second cycle is completed. At the 12^{th} week, the therapeutic results are confirmed using CT scanning. Using the WHO solid tumor objective evaluation standards, we calculated the cancer reduction rate (%) at points corresponding to 40Gy, 70Gy, and the time of confirmation of the therapeutic results. The therapeutic results are classified as Complete Response (CR), Partial Response (PR), Stable Disease (SD), and Progressive Disease (PD).

### 2. Group of Gene Therapy group combined with Chemotherapy

Gendicine was injected inside of the tumor one dosage per week. The course of whole treatment is 8 weeks, and 8 injections are performed. Chemotherapy starts three days after Gendicine injection. DDP (Cis-Platinum) of 80mg/m² was intravenously injected on Day 1. From Day 1 to Day 5, 5-FU (5-Fluororacil) was continuously injected intravenously at a dosage of 500 mg/m².

### 3. Clinical lab testing:

Regular testing of the patients begins within one week after patient is admitted into the clinical trial, and weekly after the start of the clinical trial. The testing items included physical examination, KPS scoring, complete blood test, complete urine test, and complete stool test. Every month, a biochemical test including Blood Urea Nitrogen (BUN), Creatinine (Cr), AST and ALT, EKG and chest X-ray are performed. Each week, we used the WHO classification standards for evaluating acute and sub-acute toxicity for anticancer drugs to determine and record the toxicity of Gendicine in these trials. The categories are light (I), medium (II), serious (III), and Life-threatening (IV). According to the testing results of the Phase I clinical results, changes in body temperature was especially noted.

### 4. Results analysis, and statistical analysis

All statistical analysis was performed using the SPSS11.0 statistics software. The testing was done according to ITT testing method. Comparison of the Reduction of the size of the tumor lesion was done using t test. Comparison of the therapeutic significance was done using the Pearson Chi-Square test.

### 5. Effect on Blood, Urine, Stool, and effect on liver and kidney function. The results are shown in Table 1.

**Table 1. The complete blood count and blood biochemistry testing of GT/RT Testing Group with 37 subjects.**

| Items | Pre-injection | 4 weeks | 8 weeks | P Value | |
|---|---|---|---|---|---|
| | (A) | (B) | (C) | (A-B) | (A-C) |
| WBC (10⁹/L) | 6.81±2.10 | 5.48±1.47 | 5.41±1.58 | 0.003^{▲} | 0.002^{▲} |
| Hemoglobin (g/L) | 134.05±15.08 | 126.03±14.73 | 127.28±14.51 | 0.040^{▲} | 0.095 |
| Plt (10⁹/L) | 250.49±69.67 | 242.19±86.74 | 250.58±110.33 | 0.893 | 1.000 |
| AST (U/L) | 27.59±13.70 | 25.51±12.82 | 26.08±11.16 | 0.701 | 0.829 |
| ALT (U/L) | 31.14±29.39 | 27.84±28.44 | 26.76±27.06 | 0.835 | 0.734 |
| BUN (µmol/L) | 5.51±1.12 | 4.83±1.21 | 4.80±1.66 | 0.046^{▲} | 0.110 |
| Cr (µmol/L) | 80.59±12.37 | 78.64±11.21 | 77.53±11.41 | 0.698 | 0.429 |

| | | | | | |
|---|---|---|---|---|---|
| ^{▲} shows that there are differences between the two compared groups (P<0.05) | | | | | |

The results in Table 1 indicate that, as compared between pre-injection of Gendicine, and two weeks after Gendicine injection, Complete Blood Count and Blood Biochemistry testing were all within normal range. Before injection of Gendicine, ALT, AST, BUN and Cr value were normal, and after two weeks of Gendicine injection, their values were only slightly decreased. Chest X-ray and EKG showed no changes before and after Gendicine injection.

Example Three. Gendicine therapy combined with Chemotherapy for Liver Cancer Patients.

Human recombinant p53 adenovirus injection (Gendicine) was prepared as described in Example One.

This clinical trial was designed as single-center, concurrent control, single-agent, open label, randomized clinical trial.

The patient selection standards were as follows: First, they signed consent agreement to participate in the clinical trial. Pathological and histological tests confirmed that they had suffered from Hepatocellular carcinoma (HCC). The evaluation standards for HCC satisfied the Chinese Standard Protocols for the Diagnosis and Treatment of Common Malignancies. The tumor must possess measurable lesions that were easily observed and can be injected locally with drug injection. Their ages ranged from 18 to 75, and their life expectancy was at least 3 months. Both male and female patients may be admitted.

The exclusion criteria were as follows: Patients suffered from acute upper respiratory infection, or those whose having uncontrolled fever derived from such infection are excluded. Patients were also excluded if the lesions observed were being treated with other locally-administered medication. Those patients with serious heart, liver, lung, or kidney diseases, tendency of hemorrhage, patients who were pregnant women or nursing mothers were also excluded.

The withdraw criteria was as follows: Patients who could not bear the high fever caused by using the tested medicine, patients who must stop the trial because of the onset of unexpected toxic side effects, and those patients who withdrew for personal reasons halfway were withdrew from the trials.

From March 2004 to July 2004, there were 35 cases in the liver cancer clinical trial of Gendicine Gene Therapy and chemotherapy combination therapy.

### 1). Therapeutic Protocols:

There were altogether 75 HCC patients in this clinical trial, with 51 males, and 24 females. The HCC cancer patients were at mid to late stages.

Two groups were randomly established.

The first group of 40 patients went through simple chemotherapy, using trans-catheter hepatic arterial chemo-embolization (TACE). Using routine femoral artery puncture, the catheter was implanted into celiac artery, or common hepatic artery. Afterwards, superior mesenteric arterial or phrenic artery photography was used to determine tumor number, location, type, size, supply blood vessels, and arteriovenous fistulas, thus identify other supply arteries for liver cancer. We used gelatin sponge embolism and injected 5-fluoracil (5-FU), Adriamycin (ADM), Mitomycin (MMC), cisplatin and Hydroxycamptothecin (DDP/HCPT) into the hepatic artery, in order to execute chemotherapy. After that, we introduced catheter super-selectively into the blood supplying branches of the hepatic artery for tumor, and injected the completely emulsified mixture of 10mg ADM and 10-30ml Iodinate Oil into the liver slowly under the monitor of X-ray. TACE was injected every 4 weeks and the therapeutic effects were evaluated after two injections.

The second group of 35 patients went through the Gene Therapy combined with TACE (GT-TACE). 48-72 hours before the TACE, Gendicine was injected under the guidance of CT using percutaneous intratumor injection at multiple spots in the tumor. Based upon the size of the tumor, the dosage was about 1-4x10¹² VP each time, once a week, 3-4 injections continuously. The TACE chemotherapy was administered as described above.

According to the WHO solid tumor objective evaluation standards, the therapeutic results are classified as Complete Response (CR), Partial Response (PR), Stable Disease (SD), and Progressive Disease (PD). 2). Routine standard clinical testing.

Regular testing of the patients begins within one week after patient is admitted into the clinical trial, and weekly after the start of the clinical trial. The testing items included physical examination, KPS (Karnofsky Performance Status) scoring, complete blood test, complete urine test, and complete stool test. Every month, a biochemical test including Blood Urea Nitrogen (BUN), Creatinine (Cr), AST and ALT, EKG and chest X-ray are performed. Each week, we used the WHO classification standards for evaluating acute and sub-acute toxicity for anticancer drugs to determine and record the toxicity of Gendicine in these trials. The categories are light (I), medium (II), serious (III), and Life-critical (IV). According to the testing results of the Phase I clinical results, changes in body temperature was especially noted. The general condition of patients is scored using the KPS methodology.

### 3). Results analysis and statistical analysis.

All data were analyzed using the SPSS 11.0 statistical software.

### 4). Effect on white blood cells count, symptoms, and KPS scores.

The effect on white blood cells is shown in Table 2. It can be seen that the white blood cell count is decreased significantly in control group of simple TACE chemotherapy. The difference between the two groups was significant, with p<0.05.

**Table 2. Extent of leukocytopenia and Number of Cases.**

| Groups | White Blood Cell count (x 10⁹/L) | | | Total Number of Cases showing Reduction |
|---|---|---|---|---|
| | 4.0-3.0 | 3.0-2.0 | 2.0 or lower | |
| GT-TACE | 12 | 4 | 2 | 18 |
| TACE | 8 | 20 | 11 | 39 |

Tables 3 shows the results in the improvement of the symptoms of the patients. From Table 3, it can be seen that after one month of treatment, the GT-TACE group showed drastic improvement of symptoms. The difference between two groups was significant, with p<0.05.

**Table 3. Improvement of patients after one month of therapy**

| Groups | Number of Cases | Fever | Digestive Reaction | Reduced Pain |
|---|---|---|---|---|
| GT-TACE | 35 | 25 (71.4) | 16(45.6) | 30 (85.7) |
| TACE | 40 | 18 (45.0) | 24 (60.0) | 21 (52.5) |

Table 4 shows the improvement of the patients in terms of their KPS scoring. It can be seen from Table 4 that, after one month of treatment, the GT-TACE group of patients shows significant improvement in terms of its KPS scoring.

**Table 4. KPS Scoring changes after one month of therapy**

| Group | Cases | Increase 20 | Increase 10 | No change | Reduced | Total of Increased |
|---|---|---|---|---|---|---|
| GT-TACE | 35 | 7 | 15 | 9 | 4 | 22 (62.9) |
| TACE | 40 | 9 | 10 | 8 | 13 | 19 (47.5) |

The results in this Example demonstrate that the patients in group of Gene therapy combined with chemotherapy (GT-TACE) exhibited more improvements in reducing the tumor size of patients, enhancing the symptoms, the change of white blood count and KPS scoring, as compared to the control group of chemotherapy alone. In addition, the appetite of the patients and the sleeping improved a great deal in GT-TACE group. These results indicate that when human recombinant p53 adenovirus product (Gendicine) was used to treat liver cancer in combination with chemotherapy, Gendicine can alleviate the side effects caused by chemotherapy alone and can also improve the general condition and mental status of patients.

### Example Four: The efficacy of Gendicine alone in improving the life quality of tumor patients.

A 40-year old woman suffered from a left advanced breast cancer had previously subjected to surgery. Surgical approach includes mastectomy and axillary lymphnode dissection. After the surgery, it was discovered that, 10 out of 12 lymph nodes removed surgically had cancer cell metastases. Later on, combination of radiotherapy at the dose level of 45Gy/25f/5w, and 9 cycles of chemotherapy of Ciclofosfamid plus Erubicin plus 5-fluoracil was performed on the patient. However, six month after the combination therapy, metastasized tumor was found in multiple spine regions including cervical vertebra, thoracic vertebra, and lumbar vertebra. Additional treatment of chemotherapy with Taxotere, Vinoralbine, and a new oral chemotherapy drug Xeloda, was performed on the patient. During the intermission periods of chemotherapy, the patient used Zometa to maintain bone stability. But, all of the above treatments failed to achieve the desired therapeutic effect. By January 2004, the patient showed increased level of ALT and AST, and blood count. Her physical condition started to deteriorate, and she started to feel body aches, fatigue, weight loss, mental depression, etc, indicating her condition had advanced.

After failing to achieve effective effect by chemotherapy and radiotherapy in her local hospital, the patient undertook one treatment of Gendicine therapy in hospital. The patient then brought home Gendicine to continue the therapy, and has reported back her progress in the therapy. One month following the injection of Gendicine, CT and MRI tests suggested that the tumor advanced in past six months was under control. The patient believed that it is a promising sign, "I felt very tired on the day of injection. However, I felt better and vigorous without any pain in days before the next injection." 3 months after the treatment of Gendicine, an MRI scanning was conducted, which showed that steatosis was found at the lesions of thoracic vertebra 11, 12 and lumbar vertebra 3. No advancements were observed in other metastases. The results of blood test were normal and the level of bilirubin was appreciably high. She felt no back pain and had good appetite. At 5 months after treated with Gendicine, the patient went vacation with her family and did not feel any uncomfortable and had a good metal status. After the treatment of Gendicine for 7 months, the result of MRI scanning suggested that the tumor at thoracic vertebra 11 had disappeared and no advancement was observed in other lesions without new lesions observed. There was no opacity observed in the chest X-ray. The blood count reached completely to a normal level. The patient felt no pain and had a good mental status. After the treatment of Gendicine for 9 months, the result of MRI scanning suggested that there was no sign of tumor deterioration. Results of pulmonary X-ray were good. The blood count reached to normal level. The patient felt no pain and had a better self-sensation than before.

### Example 5

A 60-year old woman was diagnosed as an invasive pancreatic cancer with a tumor having a diameter of 5 cm and was expected to have a life expectancy of only one year. She decided not to receive the routine chemotherapy because she could not burden the side effects associated with the chemotherapy. Instead, she was subjected to Gendicine treatment and injected 20 doses in 8 weeks with intravenous injection of Gendicine.

After the treatment of Gendicine, CT Scanning confirmed that the tumor in her pancreas did not advance and small injuries on her liver disappeared. After further observation, it was found that the lesions became stable, the suspicious injuries disappeared, and the swollen lymph nodes shrank.

### Conclusion

We have found that recombinant adenovirus expressingp53 has a surprisingly unique effect when it is injected into tumor patients. That is, when combined with chemotherapy or radiotherapy, it can alleviate the side effects caused by treating tumors with radiotherapy or chemotherapy alone.

This discovery is significant because after the injection of Gendicine, tumor patients, especially those in late stage, the general condition of patients have significantly improved. For example, their psychological status and mental status became better and their appetite improved. We postulate that this effect is due to Gendicine's global regulation of the nerve-endocrine-immune system. Thus, Gendicine is able to improve and strengthen the function of various organs of patients, and improve their health overall.

The possible mechanism is described as follows:

Gendicine is a viral particle manufactured by genetic engineering. It can stimulate and modulate the body's nervous system, endocrine system, and immune system to produce a series of nerve factors, hormones, and cellular factors. As seen from the examples above, one phenomenon experienced by patients after administration of Gendicine is the elevated body temperature, in other words, fever. This phenomenon can be explained that Gendicine may activate the immune system of patients. It appears that through the global regulation of the nerve-endocrine-immune system network, the patient's immune capability is improved and effectively increase the capability of NK cells and CTL cells of killing tumor cells, thus improving the anti-tumor effect of humoral immunity and cell immunity. Gendicine can also act to regulate physiological function of the patient, and therefore improve the patient's overall health.

Although the foregoing invention has been described in some detail by way of illustration and example for purposes of clarity of understanding regarding the new finding for the therapeutic effects of Gendicine, it will be apparent to those skilled in the art that certain changes and modifications will be practiced. Therefore, the description and examples should not be construed as limiting the scope of the invention, which is defined by the claims.

## Claims

1. Use of a treatment effective amount of a recombinant adenovirus expressing p53 for the preparation of a pharmaceutical composition for
a) ameliorating pain, Karnofsky Performance Status scoring or leukocytopenia caused by anti-tumor chemotherapy; or
b) ameliorating leukocytopenia caused by anti-tumor radiotherapy.

2. The use of claim 1, wherein a single dosage of said pharmaceutical composition is to be administered prior to the administration of the chemotherapy or radiotherapy.

3. The use of claim 2, wherein the single dosage of said pharmaceutical composition is to be administered on any day of the 3 days preceding the day of administration of the chemotherapy or radiotherapy.

4. The use of claim 3, wherein the single dosage of said pharmaceutical composition is to be administered on the day before the day of administration of the chemotherapy or radiotherapy.

5. The use of claim 1, wherein a single dosage of said pharmaceutical composition is to be administered on the day of administration of the chemotherapy or radiotherapy.

6. The use of claim 5, wherein the single dosage of said pharmaceutical composition is to be administered simultaneously with the chemotherapy or radiotherapy.

## Patentansprüche

1. Verwendung einer therapeutisch wirksamen Menge eines p53-exprimierenden rekombinanten Adenovirus für die Herstellung einer pharmazeutischen Zusammensetzung zur
(a) Verbesserung von Schmerz, Karnofsky Performance Status Scoring oder Leukozytopenie, verursacht durch Anti-Tumor-Chemotherapie; oder
(b) Verbesserung von Leukozytopenie verursacht durch Anti-Tumor-Radiotherapie.

2. Verwendung nach Anspruch 1, wobei eine einzelne Dosis der pharmazeutischen Zusammensetzung vor der Verabreichung der Chemotherapie oder Radiotherapie zu verabreichen ist.

3. Verwendung nach Anspruch 2, wobei die einzelne Dosis der pharmazeutischen Zusammensetzung an irgendeinem Tag der drei Tage, die dem Tag der Verabreichung der Chemotherapie oder Radiotherapie vorangehen, zu verabreichen ist.

4. Verwendung nach Anspruch 3, wobei die einzelne Dosis der pharmazeutischen Zusammensetzung am Tag vor dem Tag der Verabreichung der Chemotherapie oder Radiotherapie zu verabreichen ist.

5. Verwendung nach Anspruch 1, wobei eine einzelne Dosis der pharmazeutischen Zusammensetzung am Tag der Verabreichung der Chemotherapie oder Radiotherapie zu verabreichen ist.

6. Verwendung nach Anspruch 5, wobei die einzelne Dosis der pharmazeutischen Zusammensetzung gleichzeitig mit der Chemotherapie oder Radiotherapie zu verabreichen ist.

## Revendications

1. Utilisation d'une quantité, efficace pour un traitement, d'un adénovirus recombiné exprimant p53, pour la préparation d'une composition pharmaceutique pour
a) améliorer une douleur, un score de performance de Karnofsky ou une leucocytopénie provoquée par une chimiothérapie antitumorale; ou
b) améliorer une leucocytopénie provoquée par une radiothérapie antitumorale.

2. Utilisation selon la revendication 1, dans laquelle une dose unique de ladite composition pharmaceutique doit être administrée avant l'administration de la chimiothérapie ou radiothérapie.

3. Utilisation selon la revendication 2, dans laquelle la dose unique de ladite composition pharmaceutique doit être administrée n'importe quel jour parmi les 3 jours précédant le jour de l'administration de la chimiothérapie ou radiothérapie.

4. Utilisation selon la revendication 3, dans laquelle la dose unique de ladite composition pharmaceutique doit être administrée le jour précédant le jour de l'administration de la chimiothérapie ou radiothérapie.

5. Utilisation selon la revendication 1, dans laquelle une dose unique de ladite composition pharmaceutique doit être administrée le jour de l'administration de la chimiothérapie ou radiothérapie.

6. Utilisation selon la revendication 5, dans laquelle la dose unique de ladite composition pharmaceutique doit être administrée en même temps que la chimiothérapie ou radiothérapie.
